# EUROPEAN PATENT APPLICATION

(11) **EP 4 775 589 A1**
(43) Date of publication of application: **15.07.2026**
(21) Application number: 23951059.7
(22) Date of filing: 08.09.2023
(51) Int. Cl.: C07K 7/14, A61K 38/10, A61P 9/08, A61P 9/12

(54) **PHARMACEUTICAL COMPOSITION COMPRISING A PEPTIDE OR A COMBINATION OF PEPTIDES AGONISTIC TO THE MAS, AT1 AND AT2 RECEPTORS**

(71) Applicant: Pontificia Universidad Católica de Chile, Santiago (CL); Universidad De Chile, Santiago (CL); Universidad Andrés Bello, Santiago (CL)
(72) Inventor: OCARANZA JERALDINO, María Paz Alejandra, Providencia Santiago (CL); JALIL MILAD, Jorge Emilio, Las Condes Santiago (CL); GONZÁLEZ NILO, Fernando Danilo, Colina Santiago (CL); MORALES MONTECINOS, Javier Octavio, Independencia Santiago (CL); OYARZÚN AMPUERO, Felipe Andres, Independencia Santiago (CL); SOTO BUSTAMANTE, Eduardo Arturo, Independencia Santiago (CL)
(74) Representative: Balder IP Law, S.L.
(86) International application number: PCT/CL2023/050084
(87) International publication number: WO 2025/050228

(57) **Abstract**

The present invention relates to an agonist peptide of the Mas, AT1, and AT2 receptors, having amino acid sequence SEQ ID NO: 1, which is useful in preventing and/or treating hypertension, and/or reducing cardiovascular, cerebral, pulmonary and renal vasoconstriction, and/or preventing, reversing, inhibiting, and/or reducing the pathological remodeling of cardiovascular, pulmonary, renal and/or cerebral tissue. The invention also relates to a pharmaceutical composition comprising the peptide or a combination of the peptides, useful for preventing and/or treating hypertension, reducing vascular vasoconstriction, and/or preventing, reversing, inhibiting, and/or reducing the pathological remodeling of cardiovascular, pulmonary, renal, and/or cerebral tissue.

## Description

### TECHNICAL FIELD

The present invention relates to the technical field of the pharmaceutical industry, in particular to agonist peptides of the Mas, AT1, and AT2 receptors, and a pharmaceutical composition comprising one or a combination of said peptides useful for preventing and/or treating hypertension, reducing vascular vasoconstriction, and/or preventing, reversing, inhibiting, and/or reducing the pathological remodeling of cardiovascular, pulmonary, renal, and/or cerebral tissues.

### BACKGROUND

The renin-angiotensin system (RAS) is a hormonal system that regulates blood pressure and electrolyte homeostasis, and is composed of the angiotensin-I-converting enzyme (ACE) and its product angiotensin II, which exerts its effects through binding to AT1 and AT2 receptors. Overstimulation of the RAS has pathophysiological effects such as hypertension, atherosclerosis, heart failure, coronary heart disease, etc.

There is a parallel RAS pathway mediated by the homologous angiotensin-I-converting enzyme (ACE2) that competes with ACE for the hydrolysis of angiotensin I to form angiotensin-(1-9). Subsequently, angiotensin-(1-9) is degraded by ACE to angiotensin-(1-7). Angiotensin-(1-7) is a peptide with properties that antagonize the effects of angiotensin II: it induces vasodilation and has antihypertensive and antifibrotic effects (Padda RS, et al. Angiotensin-(1-7): A Novel Peptide to Treat Hypertension and Nephropathy in Diabetes? J Diabetes Metab. 2015. Vol. 6(10)). Likewise, the Mas receptor is a protein found on the surface of cells in many tissues of the body, including the heart and blood vessels. This receptor is an integral part of the RAS and plays an important role in regulating blood pressure through its interaction with angiotensin-(1-7). Angiotensin-(1-7) binds to the Mas receptor, triggering a cascade that ultimately produces vasodilation (Santos, R. A., Simoes e Silva, A. C., Maric, C., Silva, D. M., Machado, R. P., de Buhr, I., ... & Campagnole-Santos, M. J. Angiotensin-(1-7) is an endogenous ligand for the G protein-coupled receptor Mas. Proc Natl Acad Sci. 2003. Vol. 100(14):8258-8263).

In turn, angiotensin-(1-9) also has effects opposite to those of angiotensin II: it lowers blood pressure and reduces cardiovascular damage. Chronic administration of angiotensin-(1-9) to hypertensive rats has been shown to reduce systolic and diastolic blood pressure, improve cardiac and endothelial function, as well as cardiovascular remodeling and oxidative stress (Ocaranza MP, et al. Angiotensin-(1-9) reverses experimental hypertension and cardiovascular damage by inhibition of the angiotensin converting enzyme/Ang II axis. J Hypertens. 2014. 32(4):771-83). Also, the administration of angiotensin-(1-9) using osmotic minipumps to rats infarcted by left coronary artery ligation reduced cardiac hypertrophy, as assessed by decreased levels of ANF (atrial natriuretic factor) mRNA, β-MHC (beta-myosin heavy chain) protein levels, and cardiac myocyte size (area and perimeter) (Ocaranza MP, et al. Angiotensin-(1-9) regulates cardiac hypertrophy in vivo and in vitro. J Hypertens. 2010. 28(5):1054-64).

Although peptides have high bioactivity and specificity with few or no side effects, as is the case with angiotensin-(1-9), most peptides are short-lived molecules because they are easily degraded by enzymes, with little or no therapeutic use. The high number of peptidases present in the blood makes their half-life in plasma is very short. One strategy for designing peptides that are stable against protease degradation is the synthesis of retro-enantio analogues, also called retro-inverso analogues, which consists of incorporating non-natural D-amino acids, unlike the original peptide composed of L-amino acids, and also reversing the order of the amino acids with respect to the original sequence, thus theoretically resulting in a peptide that presents a side chain orientation very similar to that of the original structure (Van Regenmortel MHV & Muller S. D-peptides as immunogens and diagnostic reagents. Curr Opin Biotechnol. 1998. Vol. 9(4):377-382). For example, patent application WO2022238734A1 discloses the synthesis and use of an angiotensin-(1-9) corresponding to a retro-enantio of angiotensin-(1-9), which has greater stability and plasma half-life than native angiotensin-(1-9) and, in turn, maintains the same biological activity of the peptide composed of L-amino acids.

Although the antihypertensive effects of the retro-enantio peptide of angiotensin-(1-9) have been demonstrated, this may not be sufficient, considering that most patients with hypertension need a combination of at least two conventional drugs to normalize their blood pressure levels, and between 15-20% of patients require a combination of at least three drugs.

This background has given rise to the need to find alternatives to classic antihypertensive treatments, developing dual or triple inhibitors, i.e., with the aim of inhibiting enzymes involved in the generation of vasoconstrictor peptides or acting as blockers of receptors that trigger vasoconstrictor mechanisms.

### SUMMARY OF THE INVENTION

A first object of the present invention corresponds to an agonist peptide of the Mas, AT1, and AT2 receptors, comprising the amino acid sequence ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1). Said peptide is used for preventing and/or treating hypertension, and/or reducing vasoconstriction at the cardiovascular, cerebral, pulmonary, and renal levels, and/or preventing, reversing, inhibiting, and/or reducing pathological remodeling of cardiovascular, pulmonary, renal, and/or cerebral tissues, including, but not limited to heart failure with preserved, mildly reduced, or reduced ejection fraction (congestive heart failure, compensated heart failure, decompensated heart failure, and the like), restenosis of coronary arteries and other arteries with atherosclerosis, arterial hypertension (essential hypertension, low-renin hypertension, salt-sensitive hypertension, low-renin salt-sensitive hypertension, resistant and refractory arterial hypertension, pregnancy-induced hypertension; primary pulmonary hypertension, thromboembolic pulmonary hypertension; renovascular hypertension), cardiac hypertrophy, diastolic dysfunction, coronary artery disease, myocardial infarctions, cerebral infarctions, atherosclerosis, atherogenesis, cerebrovascular disease, angina (including chronic, stable, unstable, and variant angina pectoris), aneurysm, ischemic heart disease, cerebral ischemia, myocardial ischemia, thrombosis, platelet aggregation, platelet adhesion, smooth muscle cell proliferation, vascular or non-vascular complications associated with the use of medical devices, wounds associated with the use of medical devices, vascular or non-vascular wall damage, peripheral vascular disease, neointimal hyperplasia after percutaneous transluminal coronary angiography, vascular grafting, arterial bypass or coronary bypass surgery, thromboembolic events, post-angioplasty restenosis, coronary plaque inflammation, hypercholesterolemia, embolism, stroke, shock, arrhythmia, atrial fibrillation or atrial flutter, thrombotic occlusion, and cerebrovascular reclusion.

Since this invention has anti-inflammatory properties, this peptide is used for preventing and/or treating systemic and more localized diseases with inflammatory pathogenesis, such as autoimmune and degenerative diseases with an inflammatory component (lupus erythematosus, rheumatoid arthritis, polymyositis, dermatomyositis, polymyalgia rheumatica, localized or systemic osteoarthritis). It is also used for liver diseases with inflammatory and fibrotic pathogenesis such as fatty liver or hepatic steatosis of different etiologies, chronic liver damage of different etiologies, hepatic cirrhosis of different etiologies, for respiratory diseases with inflammatory and fibrotic pathogenesis such as pulmonary fibrosis and/or chronic lung damage of different etiologies, for intestinal or digestive diseases with inflammatory and fibrotic pathogenesis such as Crohn's disease, ulcerative colitis, colonic diverticulosis, plastic peritonitis.

A second object of the present invention corresponds to a pharmaceutical composition comprising an agonist peptide of the Mas, AT1, and AT2 receptors that includes the amino acid sequence ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) and a pharmaceutically acceptable excipient; or a pharmaceutical composition comprising a combination of Mas, AT1, and AT2 receptors agonist peptides whose amino acid sequences include the amino acid sequence ^{D}H^{D}F^{D}P^{D}H^{D}I^{D}Y^{D}V^{D}R^{D}D (SEQ ID NO: 2) and the amino acid sequence ^{L}D^{L}R^{L}V^{L}Y^{L}I^{L}H^{L}P (SEQ ID NO: 3) and a pharmaceutically acceptable excipient.

In a preferred embodiment of the present invention, the pharmaceutical composition includes the peptide of SEQ ID NO: 1 in a concentration between 10⁻¹⁸ M and 10⁻⁴ M.

In a preferred embodiment of the present invention, any of the pharmaceutical compositions described above may additionally include angiotensin-I-converting enzyme inhibitors, angiotensin II receptor antagonists, Rho kinase inhibitors, diuretics, calcium channel blockers, renin inhibitors, beta-blockers, neprilysin inhibitors, dual angiotensin II receptor and neprilysin antagonists, other retro-inverso peptides, and other cardiovascular drugs such as SGLT2 inhibitors (used in the treatment of heart failure and diabetes) and myosin inhibitors (used in the treatment of hypertrophic cardiomyopathy). Herein, angiotensin-I-converting enzyme inhibitors include, but are not limited to, lisinopril, enalapril, captopril, zofenopril, ramipril, quinapril, perindopril, benazepril, and fosinopril. Herein, angiotensin II receptor antagonists include, but are not limited to, valsartan, telmisartan, losartan, irbesartan, olmesartan, candesartan, eprosartan, and saralasin. Herein, calcium channel blockers include, but are not limited to, dihydropyridines (nicardipine, nifedipine, amlodipine, felodipine, nitrendipine, nisoldipine, isradipine, nimodipine), benzothiazepines (diltiazem, clentiazem), and phenylalkylamines (verapamil, gallopamil, anipamil, RO5967, falipamil). In this document, Rho kinase inhibitors include, but are not limited to, fasudil, hydroxyfasudil, 3-(4-Pyridyl)-1H-indole, (S) (+) 2 Methyl 1 [(4 methyl-5-20 isoquinolinyl) sulfonyl] homopiperazine, N (4 Pyridyl) N' (2,4,6-trichlorophenyl) urea. In this document, diuretics include, but are not limited to, thiazide diuretics (bendroflumethiazide, bencitiazide, chlorothiazide, chlorthalidone, hydrochlorothiazide, hydroflumethiazide, indapamide, methyclothiazide, metolazone, polythiazide, quinethazone, trichloromethiazide, xipamide), loop diuretics (furosemide, torsemide, bumetanide, ethacrynic acid), carbonic anhydrase inhibitors (acetazolamide, dorzolamide), sodium channel inhibitors (amiloride, triamterene), aldosterone antagonists (spironolactone, canrenoate, eplerenone), and osmotic compounds (mannitol). Herein, renin inhibitors include, but are not limited to, pepstatin, CGP2928, remikiren, enalkiren, zankiren, aliskiren. Herein, dual angiotensin II and neprilysin antagonists include, but are not limited to, sacubitril/valsartan. Selective neprilysin inhibitors include, but are not limited to, candoxatril, dexecadotril, ecadotril, racecadotril, sacubitril, thiorphan, UK-414,495. Non-selective neprilysin inhibitors include, but are not limited to, aladotril, alatriopril, daglutril, fasidotril, gemopatrilat, ilepatril, ketalorphan, omapatrilat, phosphoramidon, RB-101, sampatrilat, spinorphan, and tynorphin. Beta blockers include, but are not limited to, acebutolol, atenolol, bisoprolol, metoprolol, nadolol, nebivolol, propranolol. SGLT2 inhibitors include, but are not limited to, empagliflozin, dapagliflozin, canagliflozin. Myosin inhibitors include, but are not limited to, mavacamten and aficamten. In the present document, other retro-inverso peptides include, but are not limited to, retro-inverso bradykinin and retro-inverso AT1R.

In another preferred embodiment of the present invention, any of the pharmaceutical compositions described above are formulated for oral, intravenous, intramuscular, intraperitoneal, or continuous pump infusion administration.

A third object of the present invention relates to the use of any of the pharmaceutical compositions or their preferred embodiments described above for preparing a drug useful for preventing and/or treating hypertension; or for preparing a drug useful for reducing vasoconstriction at the cardiovascular, cerebral, pulmonary, and renal levels; or for preparing a drug useful for preventing, reversing, inhibiting, and/or reducing pathological remodeling of cardiovascular, pulmonary, renal, and/or cerebral tissues.

A fourth object of the present invention corresponds to a method for preventing and/or treating hypertension comprising administering a therapeutically effective dose of any of the pharmaceutical compositions or their preferred embodiments described above to a subject in need thereof.

### BRIEF DESCRIPTION OF THE FIGURES

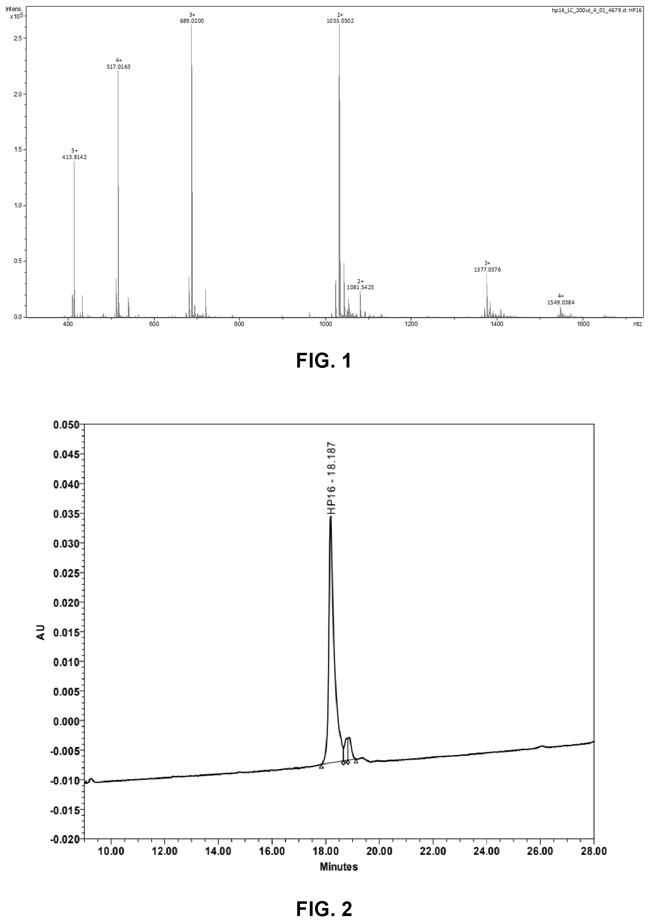

FIG. 1 shows the result of the evaluation of the molecular identity of the peptide of SEQ ID NO: 1 by mass spectrometry.
FIG. 2 shows the result of the purity determination of the peptide of SEQ ID NO: 1 (also referred to as peptide HP16) by HPLC. The graphs show the elution time of said peptide.

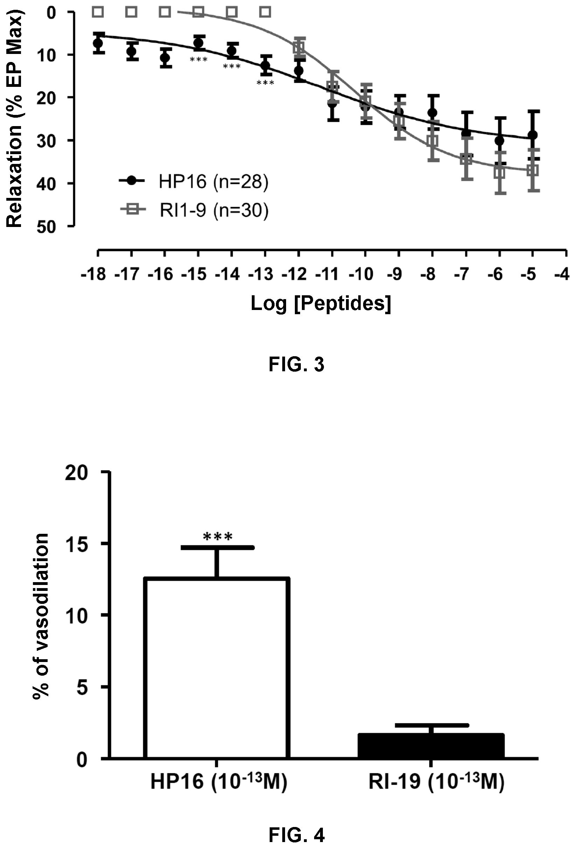

FIG. 3 shows a graph of the vasodilator effect of the angiotensin-(1-9) retro-inverso peptide (referred to as RI1-9 in the figure) and the peptide of SEQ ID NO: 1 (referred to as HP16 in the figure). Data were expressed as Mean ± SEM (n=28-30). ***p ≤ 0.001 vs. angiotensin-(1-9) retro-inverso peptide after Mann-Whitney test.
FIG. 4 shows a graph of the vasodilatory efficacy of the angiotensin-(1-9) retro-inverso peptide (referred to as RI1-9 in the figure) and peptide of SEQ ID NO: 1 (referred to as HP16 in the figure). ***p ≤ 0.001 vs. angiotensin-(1-9) retro-inverso peptide after Mann-Whitney test, n=28-30.

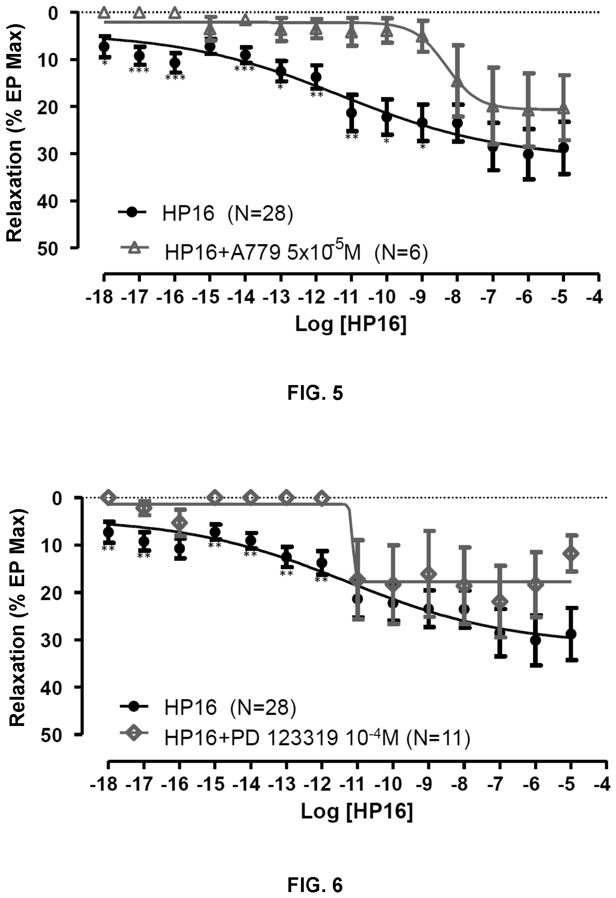

FIG. 5 shows a graph of the vasodilator effect of the peptide of SEQ ID NO: 1 (referred to as HP16 in the figure) in the presence and absence of the Mas receptor blocker for angiotensin-(1-7), A779. Data were expressed as Mean ± SEM (n=6-28). *p ≤ 0.05; **p ≤ 0.01; ***p ≤ 0.001 vs. peptide of SEQ ID NO: 1 + A779, followed by Mann-Whitney test.
FIG. 6 shows a graph of the vasodilator effect of the peptide of SEQ ID NO: 1 (referred to as HP-16 in the figure) in the presence and absence of an AT2 receptor blocker, PD123319. Data were expressed as Mean ± SEM (n=11-28). **p ≤ 0.01 vs. peptide of SEQ ID NO: 1 + PD123319, followed by Mann-Whitney test.

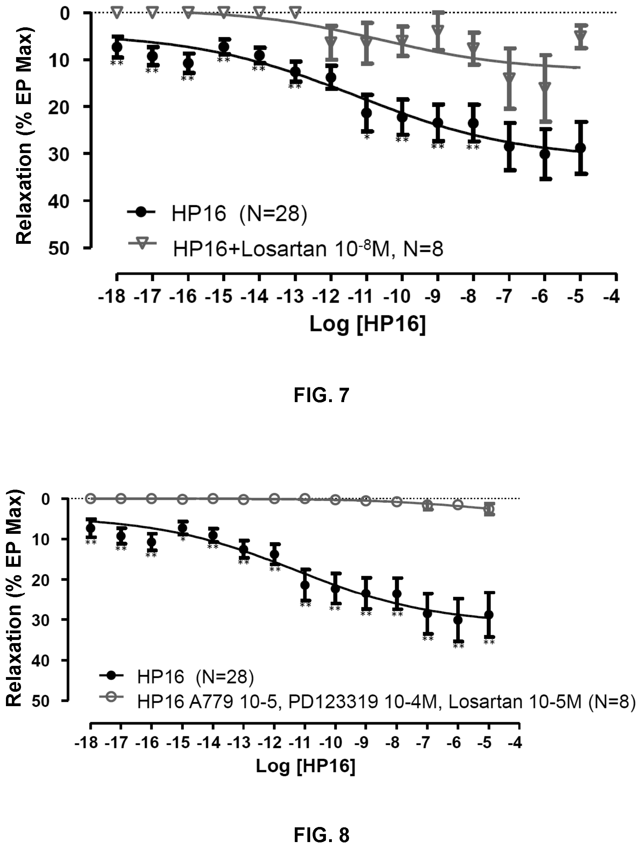

FIG. 7 shows a graph of the vasodilator effect of the peptide of SEQ ID NO: 1 (referred to as HP16 in the figure) in the presence and absence of Losartan. Data were expressed as Mean ± SEM (n=8-28). *p ≤ 0.05; **p ≤ 0.01 vs. peptide of SEQ ID NO: 1 + Losartan; *p ≤ 0.05; **p ≤ 0.01 vs. peptide of SEQ ID NO: 1, after Mann-Whitney test.
FIG. 8 shows a graph of the vasodilator effect of the peptide of SEQ ID NO: 1 (referred to as HP-16 in this figure) in the presence and absence of A779, PD123319, and Losartan. Data were expressed as Mean ± SEM (n=8-28). *p ≤ 0.05; **p ≤ 0.01 vs. peptide of SEQ ID NO: 1 + Losartan + PD123319 + A779, after Mann-Whitney test.

### DETAILED DESCRIPTION OF THE INVENTION

The present invention comprises an agonist peptide (SEQ ID NO: 1) of the Mas, AT1, and AT2 receptors and pharmaceutical compositions comprising them. This peptide, when bound to the AT1 receptor, blocks the vasoconstrictor effect and tissue damage by inducing arrestins, and when bound to the AT2 and Mas receptors, produces a vasodilator, antihypertensive, cardioprotective effect and decreases tissue damage.

All technical and scientific terms used to describe the present invention have the same meaning understood by a person with basic knowledge in the technical field in question. However, to more clearly define the scope of the invention, a list of the terminology used in this description and its meaning is included below.

The term "retro" refers to a peptide composed of D-amino acids in which the amino acids are assembled in the same direction as the native peptide.

The term "enantio" or "inverso" refers to a peptide composed of L-amino acids in which the amino acids are assembled in the opposite direction to the native peptide.

The term "retro-enantio" or "retro-inverso" refers to a peptide composed of D-amino acids in which the amino acids are assembled in the opposite direction to the native peptide.

The term "subject" should be understood to mean any mammal such as, but not limited to, humans, mice, rats, rabbits, primates, cats, dogs, among others. The term "subject" shall be understood as synonymous with the term "individual" and shall be used interchangeably in this description.

The term "treatment" is understood to mean a combination or means used for therapeutic action in a subject who has developed or is in the early stages of developing a disease, syndrome, or condition. The treatment may be a treatment of the subject's symptoms for the purpose of alleviating their symptoms, or it may refer to a treatment of the cause of the disease, syndrome, or condition for the purpose of reversing, delaying, or halting the progression of said disease, syndrome, or condition. Therefore, the pharmaceutical composition of the present invention, as well as the methods described herein, may be used, for example, as methods of therapeutic treatment for a specific period of time or on a chronic basis, as required by the subject. The term "treatment" also includes those preventive treatments that comprise treating subjects who are at risk of developing a disease, syndrome, or condition, in order to reduce said risk.

The term "effective amount" or "therapeutically effective dose" refers to the dose and period of time necessary to achieve the necessary therapeutic result. The effective amount may depend on many factors, such as the stage of the disease, age, sex, weight of the individual, presence of other diseases, simultaneous intake of other medications, race, among others.

The term "hypertension" refers to any disease or disorder associated with high blood pressure, such as low-renin hypertension, salt-sensitive hypertension, low-renin salt-sensitive hypertension, primary pulmonary hypertension, thromboembolic pulmonary hypertension, pregnancy-induced hypertension, renovascular hypertension, among others.

The term "remodeling" refers to any complex change that organs and tissues undergo when subjected to stressful conditions. This term should be understood in its broadest possible sense and involves numerous cellular, biochemical, and/or physiological processes, including hypertrophy, fibrosis, inflammation, apoptosis, necrosis, autophagy, oxidation, among others.

A first object of the present invention is an agonist peptide of the Mas, AT1, and AT2 receptors comprising or consisting of the amino acid sequence ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1), which corresponds to a fusion of the angiotensin-(1-9) retro-enantio peptide (D-amino acids, N-terminal to C-terminal direction: ^{D}H^{D}F^{D}P^{D}H^{D}I^{D}Y^{D}V^{D}R^{D}D (SEQ ID NO: 2)), and the angiotensin-(1-7) peptide (L-amino acids, N-terminal to C-terminal direction: ^{L}D^{L}R^{L}V^{L}Y^{L}I^{L}H^{L}P (SEQ ID NO: 3)). Surprisingly, the peptide of SEQ ID NO: 1 has a vasodilatory effect at a concentration 1000 times lower compared to the angiotensin-(1-9) retro-enantio peptide, and has a maximum median effective concentration (EC₅₀) in one order of magnitude lower with respect to the angiotensin-(1-9) retro-enantio peptide. In a preferred mode of the invention, the peptide of SEQ ID NO: 1 is used for preventing and/or treating hypertension, and/or for reducing vascular vasoconstriction at the cardiovascular, cerebral, pulmonary, and renal levels, and/or for preventing, reversing, inhibiting, and/or reducing pathological remodeling of cardiovascular, pulmonary, renal, and/or cerebral tissues, and/or for inducing cardioprotection.

The peptide of the present invention can be obtained by any method known in the prior art, such as, for example, by the processes described in Goodman M et al. Methods of Organic Chemistry Vol. E 22, Supplement: Synthesis of Peptides and Peptidomimetics. Georg Thieme Verlag, 2004; and Chan, W. C. Fmoc solid phase peptide synthesis: a practical approach (Vol. 222). OUP Oxford, 1999, incorporated herein by reference, and not limited to those mentioned therein. For example, peptides can be synthesized using an azide method, an acid chloride method, an acid anhydride method, a mixed anhydride method, a DCC method, an activated ester method (e.g., p-nitrophenyl ester, N-hydroxysuccinimide, or cyanomethyl ester), a carbodiimidazole method, an oxidation-reduction method, or a DCC/additive process. Said syntheses may be carried out in the solid phase and in the liquid phase. In a preferred embodiment of the present invention, the peptides are suitably prepared according to the above methods, such as those normally employed in peptide synthesis, generally by a step-by-step process that comprises condensing an amino acid in the terminal amino acid, one by one in sequence, or coupling peptide fragments to the terminal amino acid (the side groups of amino acids not used in the coupling reaction must be protected, for example with Fmoc, to prevent coupling at the wrong location).

A second object of the present invention corresponds to a pharmaceutical composition comprising an agonist peptide of the Mas, AT1, and AT2 receptors that includes the amino acid ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) and a pharmaceutically acceptable excipient.

In a preferred embodiment of the present invention, the pharmaceutical composition includes an effective amount of the peptide of SEQ ID NO: 1. Said effective amount may vary depending on multiple factors, as mentioned above. Preferably, the pharmaceutical composition includes the peptide of SEQ ID NO: 1 in a concentration between 10⁻¹⁸ M and 10⁻⁴ M, preferably between 10⁻¹⁵ M and 10⁻¹³ M.

In another preferred embodiment, the peptide (SEQ ID NO: 1) of the present invention may be administered together or in co-administration with a therapeutic agent or cardiovascular drug known in the prior art. For example, the peptide may be co-administered with angiotensin-I-converting enzyme (ACE) inhibitors, angiotensin II receptor antagonists or AT1 receptor blockers, Rho kinase inhibitors, renin inhibitors, L-type calcium channel antagonists, diuretics, and/or other retro-inverso peptides, and with other cardiovascular drugs thereof. Angiotensin-I-converting enzyme inhibitors include, but are not limited to, lisinopril, enalapril, captopril, zofenopril, ramipril, quinapril, perindopril, benazepril, and fosinopril. Angiotensin II receptor antagonists or AT1 receptor blockers include, but are not limited to, valsartan, telmisartan, losartan, irbesartan, olmesartan, candesartan, eprosartan, and saralasin. L-type calcium channel antagonists include, but are not limited to, dihydropyridines (nicardipine, nifedipine, amlodipine, felodipine, nitrendipine, nisoldipine, isradipine, nimodipine), benzothiazepines (diltiazem, clentiazem), and phenylalkylamines (verapamil, gallopamil, anipamil, RO5967, falipamil). Rho kinase inhibitors include, but are not limited to, fasudil, hydroxyfasudil, 3-(4-Pyridyl)-1H-indole, (S) (+) 2 Methyl 1 [(4 methyl-5-isoquinolinyl)sulfonyl] homopiperazine, N (4 Pyridyl) N' (2,4,6-trichlorophenyl) urea. Diuretics include, but are not limited to, thiazide diuretics (bendroflumethiazide, bencitiazide, chlorothiazide, chlorthalidone, hydrochlorothiazide, hydroflumethiazide, indapamide, methyclothiazide, metolazone, polythiazide, quinethazone, trichloromethiazide, xipamide), loop diuretics (furosemide, torsemide, bumetanide, ethacrynic acid), carbonic anhydrase inhibitors (acetazolamide, dorzolamide), sodium channel inhibitors (amiloride, triamterene), aldosterone antagonists (spironolactone, canrenoate, eplerenone), and osmotic compounds (mannitol). Renin inhibitors include, but are not limited to, pepstatin, CGP2928, remikiren, enalkiren, zankiren, and aliskiren. Here, dual angiotensin II and neprilysin antagonists include, but are not limited to, sacubitril/valsartan. Selective neprilysin inhibitors include, but are not limited to, candoxatril, dexecadotril, ecadotril, racecadotril, sacubitril, thiorphan, UK-414,495. Non-selective neprilysin inhibitors include, but are not limited to, aladotril, alatriopril, daglutril, fasidotril, gemopatrilat, ilepatril, ketalorphan, omapatrilat, phosphoramidon, RB-101, sampatrilat, spinorphan, and tynorphin. Beta-blockers include, but are not limited to, acebutolol, atenolol, bisoprolol, metoprolol, nadolol, nebivolol, and propranolol. Other retro-inverso peptides include, but are not limited to, retro-inverso bradykinin and retro-inverso AT1R.

The pharmaceutical composition of the present invention can be administered by all known routes of administration, preferably by injection and/or parenterally (e.g., intravenously, intra-arterially, intramuscularly, intraperitoneally, intradermally, subcutaneously, by direct injection into various organs, among others, without being limited to other alternatives), by inhalation, using continuous release pumps, suppositories, orally, among others, without being limited to those mentioned herein. Said administration may be in a single dose, multiple doses, or continuous administration.

The pharmaceutical composition of the present invention may be in liquid, solid, or semi-solid form, or in a mixture thereof, including tablets, wafers, coated tablets, capsules, pills, pastilles, powder, aqueous or oily suspensions, solutions, creams, ointments, coated formulations, sustained release formulations, aerosols, micro- and/or nanoparticles, among others, without being limited to those mentioned herein.

In other embodiments, the pharmaceutical compositions containing the peptide, e.g., HP-16, generally contain an effective amount of the active compound together with an appropriate amount of one or more vehicles, stabilizers, diluents, excipients, and/or adjuvants, to make possible the preparation of the appropriate dosage and form of administration of the peptide, e.g., HP-16, to the patient. In the practice of the treatment methods of the invention, the particular dosage of a pharmaceutical composition or drug to be administered to the subject will depend on many variables including the stage of the disease, the severity of the disease, the administration schedule, age, physical characteristics of the subject, etc. Appropriate doses can be established using clinical approaches as understood by those skilled in the art. The term "effective amount" refers to the dosage and period of time necessary to achieve the necessary therapeutic result, i.e., reducing tissue remodeling, increasing cardioprotection, and effectively treating cardiovascular, renal, and cerebral diseases. The effective amount may depend on many factors, such as the stage of the disease, age, sex, weight of the individual, presence of other diseases, simultaneous intake of other medications, race, among others.

In this document, the term "cardiovascular disease" refers to any cardiovascular disease or disorder known in the art, including, but not limited to, heart failure (congestive heart failure, compensated heart failure, decompensated heart failure, and the like), restenosis, hypertension (low-renin hypertension; salt-sensitive hypertension; low-renin hypertension, salt-sensitive hypertension; primary pulmonary hypertension; thromboembolic pulmonary hypertension; pregnancy-induced hypertension; renovascular hypertension), cardiac hypertrophy, diastolic dysfunction, coronary artery disease, myocardial infarctions, infarctions, atherosclerosis, atherogenesis, cerebrovascular disease, angina (including chronic, stable, unstable, and variant (Prinzmetal) angina pectoris), aneurysm, ischemic heart disease, cerebral ischemia, myocardial ischemia, thrombosis, platelet aggregation, platelet adhesion, smooth muscle cell proliferation, vascular or non-vascular complications associated with the use of medical devices, wounds associated with the use of medical devices, vascular or non-vascular wall damage, peripheral vascular disease, neointimal hyperplasia after percutaneous transluminal coronary angiography, vascular grafting, coronary artery bypass surgery, thromboembolic events, post-angioplasty restenosis, coronary plaque inflammation, hypercholesterolemia, embolism, stroke, shock, arrhythmia, atrial fibrillation or atrial flutter, thrombotic occlusion and reclusion, cerebrovascular incidents, left ventricular dysfunction and hypertrophy, and the like. One embodiment of this invention involves administering the peptide, for example, retro-inverso angiotensin-(1-9), to a patient with any of the cardiovascular diseases described above.

As used herein, the method for reducing tissue remodeling comprises reversing, inhibiting, and/or reducing cardiovascular (heart and blood vessels), pulmonary, renal, and/or cerebral remodeling in an individual or an animal. The term "remodeling" refers to the complex change that organs undergo when subjected to stressful conditions. The organs that are of particular interest in this invention for preventing remodeling through the use of retro-inverso angiotensin-(1-9) correspond to the heart, blood vessels, kidney, and brain, without excluding other organs that could undergo remodeling and that could be treated with retro-inverso angiotensin-(1-9). Remodeling should be understood in its broadest possible sense and involves numerous cellular, biochemical, and/or physiological processes, including one or all of the processes selected from cardiomyocyte hypertrophy, neointima formation, restenosis, fibroblast hyperplasia, smooth muscle cell hyperplasia, fibrosis, collagen deposition, inflammation, apoptosis, necrosis and/or autophagy, oxidation. Cardiomyocyte hypertrophy corresponds to the enlargement of cardiomyocytes, with an increase in intracellular protein content, especially those associated with the contractile machinery, and with the reexpression of fetal proteins such as β-myosin heavy chain (β-MHC) and atrial natriuretic factor (ANF). Cardiomyocyte hypertrophy is associated with cardiac hypertrophy. Cardiac hypertrophy corresponds to the growth observed in the heart in high-performance athletes (physiological or benign hypertrophy) or in hypertensive individuals or after myocardial infarction (pathological hypertrophy). Neointima formation corresponds to the formation of new, undifferentiated tissue or tissue of various types in blood vessels due to damage or any other cause, including restenosis. Restenosis is the renewed narrowing of any blood vessel, for example, the renewed narrowing of a coronary artery after angioplasty. Restenosis can be caused by many other pathologies and causes. Fibroblast hyperplasia corresponds to an increase in the number of fibroblasts due to increased proliferation. Smooth muscle cell hyperplasia corresponds to an increase in the number of smooth muscle cells due to increased proliferation. The term "fibrosis" refers to an increase in the extracellular matrix content in a tissue due to the accumulation of proteins such as collagen, fibronectin, elastin, among others. Fibrosis also involves the proliferation of fibroblasts and their differentiation into myofibroblasts. Oxidative stress is understood as an increase in reactive oxygen species and is caused by an imbalance between the synthesis and degradation of reactive oxygen species. Reactive oxygen species correspond to superoxide anion (O₃. •-), hydrogen peroxide (H₂ O₂), hydroxyl radical (OH•), and/or products of these species with other molecules that generate, for example, peroxynitrite (NOO•). Reactive oxygen species synthesis can occur in the mitochondrial oxygen transport chain, NADPH oxidase, xanthine oxidase, NO synthase, through inorganic reactions such as the Fenton reaction and the Haber-Fenton reaction, among others. The mechanisms of degradation of reactive oxygen species include natural antioxidants (e.g., vitamin C, alpha-tocopherol, uric acid, mannitol) and enzyme systems (e.g., superoxide 10 dismutase, catalase, glutathione peroxidase, among others).

In this document, the term "cardioprotection" refers to all phenomena, processes, or mechanisms involved in reducing damage or death of cardiomyocytes. Apoptosis, necrosis, and autophagy correspond to different types of cell death. In this invention, the terms hypertrophy, neointima formation, restenosis, hyperplasia, cardioprotection, fibrosis, collagen deposition, inflammation, oxidative stress, apoptosis, necrosis, and autophagy should be understood in their broadest context.

Accordingly, in additional aspects, the present invention provides a peptide (e.g., HP-16) or a pharmaceutical composition as defined above, for use in:
(i) treating a cardiovascular disease, for example, a cardiovascular disease as defined above;
(ii) preventing, reversing, inhibiting, and/or reducing cardiovascular, pulmonary, renal, and/or cerebral remodeling, preferably wherein cardiovascular remodeling comprises cardiac fibrosis, cardiomyocyte hypertrophy, cardiovascular inflammation, and/or fibroblast proliferation;
(iii) inducing cardioprotection, preferably by decreasing the size of myocardial infarction;

The pharmaceutically acceptable excipient of the present invention should be broadly understood as any component of the pharmaceutical composition other than the active compound, and may refer to a diluent, stabilizer, binder, disintegrant, coating agent, sweetener, flavoring agent, and/or coloring agent, among others, or a mixture thereof. These excipients are known in the prior art, such as those reported, for example, in Allen L, Popovich N, Ansel H. (2011). Ansel's Pharmaceutical Dosage Forms and Drug Delivery Systems. 9th edition. Lippincott Williams & Wilkins, and Rowe R, Sheskey P, Quinn M. (2009). Handbook of Pharmaceutical Excipients. 6th edition. Pharmaceutical Press, without being limited to those mentioned therein. The specific agents and compounds for each of these functions are obvious to a person skilled in the art and can be found in multiple publications such as those mentioned above.

A third object of the present invention relates to the use of any of the pharmaceutical compositions or their preferred embodiments described above for preparing a drug useful for preventing and/or treating hypertension; or for preparing a drug useful for reducing vascular vasoconstriction at the cardiovascular, cerebral, pulmonary, and renal levels; or for preparing a drug useful for preventing, reversing, inhibiting, and/or reducing pathological remodeling of cardiovascular, pulmonary, renal, and/or cerebral tissues and/or inducing cardioprotection.

A fourth object of the present invention corresponds to a method for preventing and/or treating hypertension comprising administering a therapeutically effective dose of any of the pharmaceutical compositions or their preferred embodiments described above to a subject in need thereof.

### EXAMPLES OF IMPLEMENTATION

The following examples are intended to illustrate the invention and its preferred embodiments, but in no way should they be considered to limit the scope of the invention, which will be defined by the scope of the claims attached hereto.

### Example 1. Obtaining peptides

The peptide ^{D}H^{D}F^{D}P^{D}H^{D}I^{D}Y^{D}Y^{D}R^{D}D^{L}D^{L}R^{L}Y^{L}Y^{L}I^{L}H^{L}P (SEQ ID NO: 1), which corresponds to a fusion of the angiotensin-(1-9) retro-enantio peptide (D-amino acids, N-terminal to C-terminal direction: ^{D}H^{D}F^{D}P^{D}H^{D}I^{D}Y^{D}V^{D}R^{D}D (SEQ ID NO: 2)) and the angiotensin-(1-7) peptide (L-amino acids, N-terminal to C-terminal direction: ^{L}D^{L}R^{L}V^{L}Y^{L}I^{L}H^{L}P (SEQ ID NO: 3) were synthesized by GL-Biochem (Shanghai, China).

A desired volume of solvent was taken and added to a tube containing alumina. Then, in another tube, the sieve was activated by treating it at 50°C for 15 min. Finally, the molecular sieve was dried and the solvent from the tube containing alumina was added. All solvents used were dried with alumina and molecular sieve.

Resin treatment: The required amount of resin was weighed in a reactor and washed twice with dichloromethane (DCM) after draining the reactor and adding DCM, allowing 10 min for swelling. The reactor was then drained.

Coupling of the first amino acid: 1.6 mmol of the Fmoc amino acid was weighed for each gram of resin and dissolved in the minimum possible amount of DCM. Three equivalents (relative to the amino acid) of diisopropylethylamine (DIEA) were added, stirred for 15 min, another 2 equivalents of DIEA were added, and stirred for 1-2 h. Then, 0.5 mL of methanol per gram of resin was added and stirred for 5 min. It was then washed three times with DCM, twice with dimethylformamide (DMF), and twice more with DCM, and the resin was dried.

Substitution determination: 5-10 mg of dry resin-amino acid was weighed and 1 mL of 20% piperidine (without Triton X-100) was added. It was stirred for 20 min and 30 µL of this solution was taken and diluted with 3 mL of DMF in a quartz cell. The absorbance of the samples was then measured at 290-300 nm in a 25 spectrophotometer (using DMF as a blank) and the degree of substitution was calculated using the formula (101 x A) / (7.8 x w) where A = Absorbance and w = mg of resin.

Synthesis of the second amino acid: The resin was washed with DMF. The Fmoc was removed with 20% piperidine/DMF and washed with DMF and then with DMF and DCM. The Kaiser test was performed (with a resin peptide sample). Subsequently, it was washed with DMF. Peptide cleavage was performed by acidolysis with trifluoroacetic acid (TFA), using triethylsilane and water as scavengers (94:3:3, v/v/v) for 60-90 min. The TFA was removed with a stream of N₂ and the oily residue was precipitated with dry tert-butyl ether. The crude peptide was recovered by centrifugation and decanting of the tert-butyl ether phase.

The identity was determined by mass spectrometry on a MALDI-TOF Microflex instrument (Bruker Daltonics Inc., MA-USA) in positive ion mode using linear detection (FIG. 1). The spectrum was obtained by mixing 1:1 peptide and α-cyano-4-hydroxycinnamic acid (10 mg/mL in 50% v/v acetonitrile and 0.1% v/v formic acid) on a micro scout sample plate (Bruker Daltonics Inc., MA-USA). Native Angs were used as identity controls. The spectra were acquired and visualized with a total of 250 laser impacts in the mMass version 5.5.0 program. The MALDI-TOF peptide algorithm was used to detect monoisotopic m/z signals in the spectra, using signal-to-noise ratio parameters of 3.5 and a relative intensity limit of 0.5%.

The purity of the peptides was determined by high-pressure liquid chromatography (HPLC) with a diode array detector (FIG. 2). The peptides were applied on InertSustain C18 column, particle size 5 µm. A linear gradient of 0.1% trifluoroacetic acid in acetonitrile and water was used for separation at a flow rate of 1 mL/min. A scan range of 190-700 nm was used for peptide detection.

### Example 2. Vasodilator effect of the peptide of SEQ ID NO: 1

Ten-week-old male Wistar Kyoto rats were anesthetized with ketamine (35 mg/kg) and xylazine (7 mg/kg) administered intraperitoneally. The aortas were removed, dissected from the extravascular connective tissue, and cut into 3 mm long rings in fresh Krebs buffer pH 7.4 (KRB, 120 mM NaCl, 25 mM NaHCO₃, 5 mM glucose, 4.8 mM KCI, 2.5 mM CaCl₂, 1.2 mM MgSO₄, 24 mM EDTA, and 1.2 mM KH₂PO₄) freshly bubbled with 5% CO₂ in air. They were then mounted on a wire myograph (DMT 620M) and placed in an organ bath with KRB, pH 7.4 at 37°C with constant bubbling and equilibrated for 30 min at a tension of 1.5 g. The vasodilator response of ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1), which corresponds to a fusion of the angiotensin-(1-9) retro-enantio peptide (D-amino acids, N-terminal to C-terminal direction: ^{D}H^{D}F^{D}P^{D}H^{D}I^{D}Y^{D}V^{D}R^{D}D (SEQ ID NO: 2)) and the angiotensin-(1-7) peptide (L-amino acids, N-terminal to C-terminal direction: ^{L}D^{L}R^{L}V^{L}Y^{L}I^{L}H^{L}P (SEQ ID NO: 3) were evaluated in dose-response curves [10⁻¹⁸ to 10⁻⁴ M] to these molecules in baths with aortic rings pre-contracted with phenylephrine 10⁻⁷ M. Carbacol (10⁻⁶-10⁻⁴ M) was used as a relaxation control. The peptides were added in cumulative concentrations to the baths when the vasoconstrictor response to phenylephrine reached its maximum level. Vessel tension was recorded with a PowerLab 8/25 amplifier, and the data were captured and analyzed using LabChart 7.0 software.

*Ex vivo* assays showed that ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) exerts a significantly greater vasodilatory effect at femtomolar concentrations with respect to RI-Ang-(1-9), which is at picomolar concentrations (FIG. 3).

### Example 3. Vasodilatory efficacy of the peptide of SEQ ID NO: 1

In this experiment, the percentage of vasodilation achieved by aortic rings when incubated with ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) or angiotensin-(1-9) retro-enantio (D-amino acids, N-terminal to C-terminal direction: ^{D}H^{D}F^{D}P^{D}H^{D}I^{D}Y^{D}V^{D}R^{D}D (SEQ ID NO: 2)) at a concentration of 10⁻¹³ M. ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) exerts a maximum effect of relaxation of 13.4±1.6% (FIG. 4). Meanwhile, the maximum vasodilation achieved by ^{D}H^{D}F^{D}P^{D}H^{D}I^{D}Y^{D}V^{D}R^{D}D (SEQ ID NO: 2) was 2.1±0.6% at the same concentration (FIG. 4).

### Example 4. Vasodilator effect of the peptide of SEQ ID NO: 1 in the presence and absence of A779

Aortic rings with intact endothelium were pre-incubated with Mas receptor blocker (A779, 10⁻⁷ M); then pre-contracted with phenylephrine (10⁻⁷ M) to maximum contraction, and the peptide ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) or angiotensin-(1-9) retro-enantio in increasing concentrations (10⁻¹⁸ to 10⁻⁴ M). A779 partially modified the vasorelaxation induced by ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1). This effect was evident between 10⁻¹⁸-10⁻⁹ M of ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1). At higher concentrations, A779 did not modify the vasodilation induced by the peptide ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1, FIG. 5).

### Example 5. Vasodilator effect of the peptide of SEQ ID NO: 1 in the presence and absence of PD123319

Aortic rings with intact endothelium were pre-incubated with AT2 receptor blocker (PD123319, 10⁻⁵ M) and pre-contracted with phenylephrine (10⁻⁷ M). The peptide of SEQ ID NO: 1 was added in increasing and cumulative concentrations (10⁻¹⁸ to 10⁻⁴ M). PD123319 partially blocked the vasodilator action of the peptide ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) in the range of concentrations used (FIG. 6). At concentrations greater than 10⁻¹¹ M of ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1), PD123319 did not block the vasodilator effect of ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) [FIG. 6].

### Example 6. Vasodilator effect of the peptide of SEQ ID NO: 1 in the presence and absence of Losartan

Aortic rings with intact endothelium were pre-incubated with and without the AT1 receptor blocker (Losartan, 10⁻⁶ M) and pre-contracted with phenylephrine (10⁻⁷ M). The peptide of SEQ ID NO: 1 was added in increasing and cumulative concentrations (10⁻¹⁸ to 10⁻⁴ M). The AT1 receptor blocker, losartan, significantly decreased HP16 vasodilation in the concentration range of 10⁻¹⁸-10⁻⁹ M. At higher concentrations of HP-16, losartan did not modify the vasodilatory response of HP16 (FIG. 7).

### Example 7. Vasodilator effect of the peptide of SEQ ID NO: 1 in the presence and absence of Losartan, PD123319, and A779

Aortic rings with intact endothelium were pre-incubated with vehicle or with a mixture of Mas receptor blockers (A779, 10⁻⁵ M), AT1 (Losartan, 10⁻⁵ M), and AT2 (PD123319, 10⁻⁴ M) and pre-contracted with phenylephrine (10⁻⁷ M). The peptide of SEQ ID NO: 1 was added in increasing and cumulative concentrations (10⁻¹⁸ to 10⁻⁴ M). Pre-incubation with losartan, PD123319, and A779 completely blocked the vasodilator effect of ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1, FIG. 8).

### Example 8. Effective concentration of the peptide of SEQ ID NO: 1 that produces 50% of the maximum effect

The effective concentration of the peptide ^{D}H^{D}F^{D}P^{D}H^{D}I^{D}Y^{D}V^{D}R^{D}D^{L}D^{L}R^{L}V^{L}Y^{L}I^{L}H^{L}P (SEQ ID NO: 1) and the angiotensin-(1-9) retro-inverso SEQ ID NO: 2 that produces 50% of the maximum vasorelaxation effect (EC₅₀) was calculated. In particular, EC₅₀ corresponds to the concentration of the peptide at which 50% of the vasodilatory response of aortic rings pre-contracted with phenylephrine 10⁻⁷ M is achieved. The results showed that HP16 vasorelaxes at a lower concentration of 5.089 x 10⁻¹² M (Table 1) compared to RI-Ang-(1-9) [7.67 x 10⁻¹¹ M, Table 1).

**Table 1. EC₅₀ of the angiotensin-(1-9) retro-inverso peptide (RI-Ang-(1-9)) and the peptide of SEQ ID NO: 1 (HP16).**

| **Treatment** | **EC₅₀ mol/L** |
|---|---|
| Phenylephrine + RI-Ang-(1-9) | 7.67 x 10⁻¹¹ |
| Phenylephrine + HP16 | 5.089 x 10⁻¹² |

## Claims

1. An agonist peptide of the Mas, AT1, and AT2 receptors, **CHARACTERIZED in that** it comprises the amino acid sequence SEQ ID NO: 1.

2. A pharmaceutical composition, **CHARACTERIZED in that** it includes an agonist peptide of the Mas, AT1, and AT2 receptors, comprising the amino acid sequence SEQ ID NO: 1, and a pharmaceutically acceptable excipient.

3. The pharmaceutical composition according to claim 2, **CHARACTERIZED in that** the agonist peptide of the Mas, AT1, and AT2 receptors is in a concentration between 10⁻¹⁸ M and 10⁻⁴ M.

4. The pharmaceutical composition according to claim 3, **CHARACTERIZED in that** the agonist peptide of the Mas, AT1, and AT2 receptors is in a concentration between 10⁻¹⁵ M and 10⁻¹³ M.

5. The pharmaceutical composition according to any of claims 2 to 4, **CHARACTERIZED in that** it additionally comprises a drug selected from the group consisting of an angiotensin-converting enzyme (ACE) inhibitor, an AT1 receptor blocker, a Rho kinase inhibitor, a renin inhibitor, L-type calcium channel antagonists, and/or diuretics.

6. The pharmaceutical composition according to claim 5, **CHARACTERIZED in that** said angiotensin I converting enzyme inhibitor is a drug selected from the group consisting of lisinopril, enalapril, captopril, zofenopril, ramipril, quinapril, perindopril, benazepril, and fosinopril; said angiotensin II (AT1) receptor antagonist is a drug selected from the group consisting of valsartan, telmisartan, losartan, irbesartan, olmesartan, candesartan, eprosartan, and saralazine; said L-type calcium channel antagonist is a drug selected from the group consisting of nicardipine, nifedipine, amlodipine, felodipine, nitrendipine, nisoldipine, isradipine, nimodipine, diltiazem, clentiazem, verapamil, gallopamil, anipamil, RO5967, falipamil; said rho kinase inhibitor is a drug selected from the group consisting of fasudil, hydroxylfasudil, 3-(4-Pyridyl)-1H-indole, (S)-(+)-2-Methyl-1-[(4-methyl-5-isoquinolinyl)sulfonyl] homopiperazine, N-(4-Pyridyl)-N'-(2,4,6-trichlorophenyl)urea; said diuretic is a drug selected from the group consisting of bendroflumethiazide, bencothiazide, chlorothiazide, chlorthalidone, hydrochlorothiazide, hydroflumethiazide, indapamide, methylchlorothiazide, metolazone, polythiazide, quinethazone, trichlormethiazide, xipamide, furosemide, torsemide, bumetanide, ethacrynic acid, acetazolamide, dorzolamide, amiloride, triamterene, spironolactone, canrenoate, eplerenone, and mannitol; said renin inhibitor is a drug selected from the group consisting of pepstatin, CGP2928, remikiren, enalkiren, zankiren, aliskiren; and the other retro-inverso peptide is selected from the group consisting of retro-inverso bradykinin and retro-inverso AT1R.

7. The pharmaceutical composition according to any of claims 2 to 6, **CHARACTERIZED in that** it is formulated for oral, intravenous, intramuscular, intraperitoneal, or continuous infusion by pump administration.

8. Use of an agonist peptide of the Mas, AT1, and AT2 receptors according to claim 1, **CHARACTERIZED in that** it is suitable for the preparation of a drug useful for preventing and/or treating hypertension, including low-renin hypertension, salt-sensitive hypertension, low-renin salt-sensitive hypertension, primary pulmonary hypertension, thromboembolic pulmonary hypertension; pregnancy-induced hypertension; renovascular hypertension.

9. Use of an agonist peptide of the Mas, AT1, and AT2 receptors according to claim 1, **CHARACTERIZED in that** it is suitable for the preparation of a drug useful for preventing, reversing, inhibiting, and/or reducing heart failure, including congestive heart failure, compensated heart failure, decompensated heart failure, and the like), restenosis, cardiac hypertrophy, diastolic dysfunction, coronary artery disease, myocardial infarction, cerebral infarction, atherosclerosis, atherogenesis, cerebrovascular disease, angina (including chronic, stable, unstable, and variant angina pectoris), aneurysm, ischemic heart disease, cerebral ischemia, myocardial ischemia, thrombosis, platelet aggregation, platelet adhesion, smooth muscle cell proliferation, vascular or non-vascular complications associated with the use of medical devices, wounds associated with the use of medical devices, vascular or non-vascular wall damage, peripheral vascular disease, neointimal hyperplasia after percutaneous transluminal coronary angiography, vascular grafting, coronary artery bypass surgery, thromboembolic events, post-angioplasty restenosis, coronary plaque inflammation, hypercholesterolemia, embolism, stroke, shock, arrhythmia, atrial fibrillation or atrial flutter, thrombotic occlusion, and cerebrovascular reclusion.

10. Use of an agonist peptide of the Mas, AT1, and AT2 receptors according to claim 1, **CHARACTERIZED in that** it is suitable for the preparation of a drug useful for preventing, reversing, inhibiting, and/or reducing vasoconstriction.

11. Use of an agonist peptide of the Mas, AT1, and AT2 receptors according to claim 1, **CHARACTERIZED in that** it is suitable for the preparation of a drug useful for preventing, reversing, inhibiting, and/or reducing pathological remodeling of cardiovascular, pulmonary, renal, and/or cerebral tissues.
